# EUROPEAN PATENT APPLICATION

(11) **EP 1 772 733 A1**
(43) Date of publication of application: **11.04.2007**
(21) Application number: 05292101.2
(22) Date of filing: 10.10.2005
(51) Int. Cl.: G01N 33/68

(54) **Method for the differential diagnosis and the monitoring of Alzheimer-type dementia**

(71) Applicant: INSTITUT NATIONAL DE LA SANTE ET DE LA RECHERCHE MEDICALE (INSERM), 75013 Paris (FR)
(72) Inventor: Verdier, Jean-Michel, 34980 Saint-Gely-Du-Fesc (FR)
(74) Representative: Colombet, Alain André

(57) **Abstract**

The invention concerns *in vitro* methods for the differential diagnosis and the monitoring of Alzheimer-type dementia like: Alzheimer's disease, vascular dementia, Lewy's bodies disease, Pick's disease, and fronto-temporal dementia, in a patient, which method comprises determining the level of CD5 antigen-Iike expression in a biological sample of the patient.

## Description

The invention relates to a method for the differential diagnosis and the monitoring of Alzheimer disease-type dementia and to a kit useful in such a method.

Dementia are mental disorders characterized by a persistent loss of intellectual abilities and memory impairments. They constitute a major health problem in the elderly population. Among the dementia, the Alzheimer-type dementia (ATD) are mainly the Alzheimer's disease (AD), vascular dementia (VD), Lewy's bodies disease (LBD), Pick's disease (PiD) and the fronto-temporal dementia (FTD).

AD is the most common dementia. It accounts for 50-75% of the overall cases of dementia among individuals over the age of 65, and affects more than 15 million people around the world. The etiology of AD is unknown but the current hypothesis (amyloid cascade hypothesis) states that AD is initiated by accumulation and oligomerization of a peptide, called amyloid-beta peptide (Aβ), in limbic and association cortices. This accumulation consequently induces a neuronal toxicity (Zlokovic BV et al., 2005), causing death of pyramidal neurons and loss of neuronal synapses in affected brain regions. This accumulation results in microglial and astrocytic activation and subsequent inflammatory responses, altered neuronal ionic homeostasis, altered kinase/phosphatase activities leading to neurofibrillary tangles formation, and finally neuronal/synaptic dysfunction and neuronal loss. Alzheimer's disease often includes vascular dementia (VD) (Fladd D, 2005). This syndrome involves the damage of small vessels in the brain, which could be associated with cognitive decline. Various diseases, such as diabetes and high blood pressure, predispose the individual to damage to these small vessels.

LBD is a common cause of neurodegenerative dementia in older people (Mc Keith I, 2005). It has many of the clinical and pathological features of the dementia that occurs during the course of Parkinson's disease. Patients present disseminated aggregation of the synaptic protein alpha-synuclein in neurits (Lewy's bodies) leading to multiple transmitter deficits. LBD begins with dementia complicated by visual hallucinations and parkinsonian-dementia syndrome.

PiD is a progressively degenerative neurological disease affecting the frontal and temporal lobes first, with earliest symptoms showing up as changes in personality and a decline in function at home as well as work. There is a common core of symptoms among patients which may be present at different stages of the disease. These symptoms include loss of memory, lack of spontaneity, difficulty in thinking or concentrating, and disturbances of speech. The underlying pathobiology of the neuronal loss is unknown. Recently, it has been hypothesized that a focal synaptic losses that occurs in PiD as in AD may contribute to the cognitive deficits in both conditions (Lippa et al., 2004)

FTD is a degenerative disease which affects the frontal and anterior temporal lobes of the brain. It therefore differs from other dementia such as AD by affecting control, reasoning, personality, or perseveration. Patients with FTD can present with the clinical syndrome of semantic dementia due to a progressive loss of semantic knowledge or a neuropsychiatric syndrome characterized by aberrant social behaviours, although frequently both co-exist (Williams GB, et al., 2005).

Only probabilistic statements about the presence of dementia can be made in a patient. The diagnosis of dementia can only be inferred with certainty by post-mortem examination of the brain tissue. However, because the processes leading to dementia take place decades before clinical symptoms appear, the availability of an early stage diagnosis method would be crucial for an effective treatment of these diseases. Indeed, the earlier ATD is diagnosed, the earlier drugs can be administered. Therefore, a diagnosis method pinpointing the pathology at early stages would be of great interest. In addition, because all ATD show distinctive pathognomonic lesions, developing distinctive diagnosis would allow to discriminate between the different ATD.

Numerous proteins have been described as potential useful markers for ATD. Two cerebrospinal fluid biomarkers of neuropathological injury have been widely studied because they are directly related to the pathology: the beta-amyloid peptide and the tau proteins (Andreasen N and Blennow K, 2005; U.S. 6,670,137). In addition, inflammatory markers such as IL-6, lithostathine or pancreatic associated protein (PAP) (Duplan et al., 2001), melanotransferrine (or P67) have also been disclosed, as well as neuronal destruction markers such as alpha-1-antichymotrypsin (ACT), cathepsin D or AD7c-NTP phosphoprotein. Determining the levels of glyceraldehyde-derived advanced glycation end products (AGEs) in the serum or cerebrospinal fluid have further been proposed as a promising biomarker for early detection of Alzheimer's disease (Yamagishi S et al., 2005). Yamagishi et al. (2004) also suggested that the presence of neurotrophic factor pigment-epithelium derived factor (PEDF) in cerebrospinal fluid might be a marker for AD. A PEDF immunoassay for the diagnosis of AD is disclosed in international application WO 03/046566. International application WO 04/001421 describes an immunoassay of at least one protein identified from a biological fluid from patients with dementia, which would allow diagnosing and discriminating the different dementia. Among these listed proteins, one can find haptoglobin, a stress-responsive protein. Ueno I et al. (2000) had already shown an increase of haptoglobin level in the plasma of patients with dementia except those suffering from AD.

However, so far, none of these markers has proven entirely satisfactory, the main reason being the overlapping values between healthy controls and patients, or between the different ATD.

Therefore, an object of the present invention is to provide reliable biomarkers of the various ATD diseases.

The inventors have identified a new biomarker for the diagnosis and discrimination of several ATD. By 2D-electrophoresis, they have performed a thorough study of proteins present in the cerebrospinal fluid of patients with ATD. They have distinguished several populations according to their profile with regard to the level of CD5 antigen-like expression. Moreover, they showed that PEDF and haptoglobin help to further discriminate between AD, VD, FDT, LBD, and PiD. Finally, they have revealed the existence of a correlation between these markers expression and the different ATD through original statistical analysis.

The present invention thus relates to an *in vitro* method for the differential diagnosis of an Alzheimer-type dementia in a patient. This method includes the determination of the level of expression of CD5 antigen-like or any of its isoforms in a biological sample of the patient. In practice, the level of CD5 antigen-like expression in a subject showing clinical symptoms of a particular ATD, or suspected with a particular ATD, is generally compared to predetermined values, that can be the level of CD5 antigen-like expression in "control" subjects who are subjects showing clinical symptoms of another ATD, or suspected with another ATD.

The invention further provides an *in vitro* method for monitoring a patient diagnosed with an Alzheimer-type dementia, which method comprises determining the level of expression of the CD5 antigen-like or any of its isoforms, in a biological sample of a patient, and comparing it to the level of expression of said marker in a biological sample previously obtained from the same patient, wherein an increase of the level of expression is indicative that the dementia has worsened.

Another subject of the invention is a kit useful for differential diagnosis of Alzheimer-type dementia, which comprises:
(i) means for the detection of the level of expression of CD5 antigen-like or any of its isoforms and :
(ii) means for the detection of the level of expression of haptoglobin or any of its isoforms, and/or
(iii) means for the detection of the level of expression of PEDF.

### Definitions

Alzheimer-type dementia (ATD) comprises Alzheimer's disease (AD), vascular dementia (VD), Lewy's bodies disease (LBD, Pick's disease (PiD) and fronto-temporal dementia (FTD). Referring to AD also includes the vascular dementia that is clinically related to and most often associated with AD.

The "CD5 antigen-like" is a secreted protein which belongs to the scavenger receptor cysteine-rich (SRCR) family. It was disclosed in international application WO 98/39443 as an immunoregulatory polypeptide, termed Sp-alpha and is in particular represented by SEQ ID No: 2. Other names for CD5 antigen-like are CT-2, CD5 antigen-like receptor, and IgM-associated peptide (Tissot et al., 2002). The protein is expressed in various tissues, especially in spleen, thymus, bone marrow and peripheral blood leukocytes. It is associated in regulation of inflammatory responses and it is involved in the development and maintenance of the lymphoid compartment. The CD5 antigen-like was detected in plasma/serum and was thought to be involved in the homeostasis of IgM antibodies (Tissot et al., 2002) as well as in inhibition of macrophage apoptosis during bacterial infection (Joseph et al., 2004).

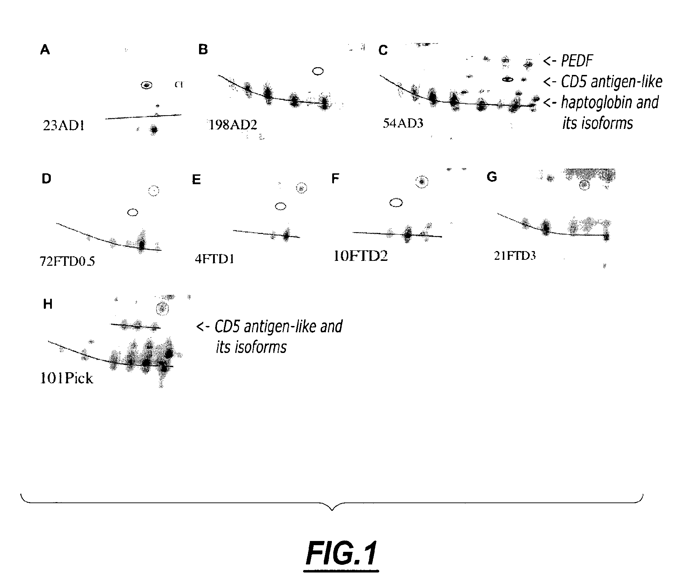

As intended herein "isoforms" of CD5 antigen-like relate to different CD5 antigen-like proteins having essentially the same molecular weight, which optionally present varying glycosylation patterns. By 2D-electrophoresis, the inventors identified several isoforms of CD5 antigen-like very useful for the differential diagnosis of the invention (cf Fig. 1H), in view of the typical arc of circle pattern formed by protein spots on the 2D gels. These isoforms exhibit essentially the same molecular weight, and represent about 50% of the total of CD5 antigen-like isoforms detected by silver nitrate staining. The isoforms most probably result from differences during the maturation process leading to distinct glycosylation patterns. It is important to notice that not all isoforms are present in all 2D gels. In other words, isoforms may be absent in several gels. Therefore, the present invention encompasses determining the level of CD5 antigen-like or any of its isoforms that are helpful to distinguish between diseases.

The haptoglobin is a stress-responsive protein. Its post-translational modifications lead to several isoforms with different glycosylation patterns depending on the tissue of expression (Smeets MB, 2003). Therefore, the present invention encompasses determining the level of haptoglobin or of any of its isoforms.

CD5 antigen-like protein, haptoglobin or PEDF are referred to as "markers" or "biomarkers", in the context of the present invention.

The term "differential diagnosis" refers to the determination or the confirmation of a disease or condition in a patient. In order to establish a diagnosis, the level of expression of a biomarker is determined in a sample of a patient with a suspicion of ATD. Said level is compared to expression levels of said marker in a biological sample from subjects with another kind of dementia. The levels of expression of these markers in other dementia diseases are thus predetermined values and constitute a range of marker levels previously defined as characteristic for samples obtained from said subjects with other dementias.

A method for "monitoring a patient" is performed in order to determine the evolution of a condition involving a dementia, for example to monitor the effect of a drug or the spreading of the condition. In this case, the patient is tested at a subsequent time, e.g. a few weeks or months after the first test. The results of the subsequent test are therefore compared with the results of the previous test. Therefore, the "control subject" herein refers to the same patient. The "previous test" refers to the first diagnosis test or to a further test, meaning that monitoring can involved as many tests as necessary.

A "biological sample" is a fluid from a subject, including serum, blood, spinal fluid, cerebrospinal fluid, urine, or a tissue extract or a tissue or organ biopsy such as brain, spinal cord or nerves. Preferably the biological sample is cerebrospinal fluid, blood or urine.
"A patient" is a vertebrate, e.g. a mammal, preferably a human being, regardless of his/her age, sex and general condition. The test patient may be asymptomatic, may be considered likely to develop the disease or condition. Subjects with a suspicion of dementia or subjects who have already shown symptoms of ATD or condition are preferred patients to be tested. Subjects who are predisposed to developing a dementia or subjects who show early symptoms of neurodegenerative impairment are a further preferred target.

### Determination of CD5 antigen-like expression

The expression of CD5 antigen-like or any of its isoforms may be determined by various techniques known by one skilled in the art. In particular it may be determined either by assaying the CD5 antigen-like protein, or by determining the level of transcription, i.e. the quantity of mRNA that encodes CD5 antigen-like. Similar procedures may be applied to determine the level of haptoglobin and PEDF expression, respectively.

Methods for assaying the CD5 antigen-like protein or any of its isoforms are well known to one skilled in the art.

Such methods comprise contacting a biological sample with a binding partner capable of selectively interacting with CD5 antigen-like present in the sample. The binding partner is generally an antibody, which may be polyclonal or monoclonal, preferably monoclonal.

Antibodies directed toward CD5 antigen-like are particularly useful. However, antibodies towards haptoglobin or any of its isoforms, and PEDF, are also of interest.

The antibodies of the present invention may be monoclonal or polyclonal antibodies, single chain or double chain, chimeric antibodies, humanized antibodies, or portions of an immunoglobulin molecule, including those portions known in the art as antigen binding fragments Fab, Fab', F(ab')₂ and F(v). As intended herein, scFv polypeptides are also considered as antibodies. They can also be immunoconjugated, e.g. with a toxin, or labelled antibodies.

Whereas polyclonal antibodies may be used, monoclonal antibodies are preferred for they are more reproducible in the long run.

Procedures for raising polyclonal antibodies are also well known. Typically, such antibodies can be raised by administering the protein or polypeptide of the present invention subcutaneously to New Zealand white rabbits which have first been bled to obtain pre-immune serum. The antigens can be injected at a total volume of 100 µl per site at six different sites. Each injected material will contain adjuvants with or without pulverized acrylamide gel containing the protein or polypeptide after SDS-polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody. This and other procedures for raising polyclonal antibodies are disclosed in E. Harlow, et. al., editors, Antibodies: A Laboratory Manual (1988).

A "monoclonal antibody" in its various grammatical forms refers to a population of antibody molecules that contain only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, e.g. a bi-specific monoclonal antibody. Although historically a monoclonal antibody was produced by immortalization of a clonally pure immunoglobulin secreting cell line, a monoclonally pure population of antibody molecules can also be prepared.

Laboratory methods for preparing monoclonal antibodies are well known in the art (see, for example, Harlow et al., Antibodies: A Laboratory Manual, Cold Spring Harbor Laboratory, New York, (1988)). Monoclonal antibodies (Mabs) may be prepared by immunizing purified CD5 antigen-like protein isolated from any of a variety of mammalian species into a mammal, e.g. a mouse, rat, rabbit, goat, human and the like mammal. The recombinant protein used for immunization can also be produced in prokaryote or eukaryote cells using an *in vitro* Gateway® type system (Invitrogen). The antibody-producing cells in the immunized mammal are isolated and fused with myeloma or heteromyeloma cells to produce hybrid cells (hybridoma). The hybridoma cells producing the monoclonal antibodies are utilized as a source of the desired monoclonal antibody.

Methods for producing antibodies as described above can be easily adapted to produce antibodies useful for the detection of CD5 antigen-like or any of its isoforms, haptoglobin or any of its isoforms and PEDF respectively useful for the method of the invention.

The preparation of anti-CD5 antigen-like monoclonal antibodies is also disclosed in WO 98/39443. A production of monoclonal and polyclonal antibodies against CD5 antigen-like is further reported in Sarrias et al. (2004).

The production of antibodies recognizing haptoglobin is disclosed in international application WO 01/75454. Anti-haptoglobin antibodies are also commercially available (for example : Goat anti-haptoglogin L15320G, Chemicon ; Sheep anti-human haptoglobin, AHP082, Serotec).

Antibodies against PEDF can be obtained as described in international application WO 03/046566 or by acquiring them for example from Chemicom Company (anti-human PEDF, MAB1059).

The presence of CD5 antigen-like can be detected using standard electrophoretic and immuno-diagnostic techniques, including immunoassays such as competition, direct reaction, or sandwich type assays. Such assays include, but are not limited to, Western blots; agglutination tests; enzyme-labelled and mediated immunoassays, such as ELISAs; biotin/avidin type assays; radio-immunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound CD5 antigen-like in a liquid phase from a solid phase support to which antigen-antibody complexes are bound. Solid supports which can be used in the practice of the invention include substrates such as nitrocellulose (e.g., in membrane or microtiter well form); polyvinylchloride (e.g., sheets or microtiter wells); polystyrene latex (e.g., beads or microtiter plates); polyvinylidine fluoride; diazotized paper; nylon membranes; activated beads, magnetically responsive beads, and the like.

Typically, a solid support is first reacted with a solid phase component (e.g., one or more anti-CD5 antigen-like antibody) under suitable binding conditions such as the component is sufficiently immobilized to the support according to methods well known to those skilled in the art. After reacting the solid support with the solid phase component, any non-immobilized solid-phase components are removed from the support by washing, and the support-bound component is then contacted with a biological sample suspected of containing ligand moieties (e.g., CD5 antigen-like molecules towards the immobilized antibodies) under suitable binding conditions. After washing to remove any nonbound ligand, a secondary binder moiety is added under suitable binding conditions, wherein the secondary binder is capable of associating selectively with the bound ligand. The presence of the secondary binder can then be detected using techniques well known in the art.

More particularly, an ELISA method can be used, wherein the wells of a microtiter plate are coated with an anti-CD5 antigen-like antibody. A biological sample containing or suspected of containing CD5 antigen-like molecules is then added to the coated wells. After a period of incubation sufficient to allow the formation of antibody-antigen complexes, the plate(s) can be washed to remove unbound moieties and a detectably labelled secondary binding molecule added. The secondary binding molecule is allowed to react with any captured sample CD5 antigen-like molecules, the plate washed and the presence of the secondary binding molecule detected using methods well known in the art.

Thus, in one particular embodiment, the presence of bound CD5 antigen-like molecules from a biological sample can be readily detected using a secondary binder comprising another antibody that can be readily conjugated to a detectable enzyme label, such as horseradish peroxidase, alkaline phosphatase or urease, using methods known to those of skill in the art. An appropriate enzyme substrate is then used to generate a detectable signal. In other related embodiments, competitive-type ELISA techniques can be practiced using methods known to those skilled in the art.

Assays can also be conducted in solution, such that the antigenic polypeptides and antibodies specific for those proteins form complexes under precipitating conditions. In one particular embodiment, antibodies can be attached to a solid phase particle (e.g., an agarose bead or the like) using coupling techniques known in the art, such as by direct chemical or indirect coupling. The antibody-coated particle is then contacted under suitable binding conditions with a biological sample suspected of containing CD5 antigen-like molecules. The particle-antigen-antibody complexes aggregate and can be precipitated and separated from the sample using washing and/or centrifugation. The reaction mixture can be analyzed to determine the presence or absence of antibody-antigen complexes using any of a number of standard methods, such as those immunodiagnostic methods described above.

Alternatively, CD5 antigen-like expression can be detected using aptamers. An aptamer is a nucleic acid that binds a wide range of biomolecules, including nucleotides, peptides, antibiotics, and cofactors. Aptamers have many advantages over antibodies as macromolecular ligands for target proteins. These advantages include small size, stability, exquisite conformational sensitivity, potential to be wholly chemically synthesised, as well as insensitivity to problems such as inter-specific sequence conservation and problems of antigen processing and presentation. Methods for producing aptamers are well known to one skilled in the art (see for example Ellington *et al.* (1990) or Wilson *et al.* (2001)).

Determining the level of expression of CD5 antigen-like protein, haptoglobin or PEDF can alternatively be performed by assaying the mRNA expression level of the respective genes.

Methods for determining the level of transcription of a gene are also well known in the art. For example the nucleic acid contained in the samples (e.g., cell or tissue prepared from the subject) is first extracted according to standard methods, for example using lytic enzymes or chemical solutions or extracted by nucleic-acid-binding resins following the manufacturer's instructions. The extracted mRNA is then detected by hybridization (e.g., Northern blot analysis) and/or amplification (e.g., PCR). Nucleic acids having at least 10 nucleotides, preferably between about 20 and 25 nucleotides, even more preferably between about 50 and 100 nucleotides, and exhibiting sequence complementarity or homology to the CD5 antigen-like mRNA herein find utility as hybridization probes. It is understood that probes need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e.g., avidin/biotin). Further, the nucleic acids can also be attached to a solid support (e.g., glass or chip) for use in high throughput screening assays using methods described, for example, in U.S. Patents. Nos. 5,405,783, 5,578,832 and 5,445,934. Results of high throughput assays can be analyzed using computer software programs available from the manufacturers.

### Kits

The invention also relates to a kit useful for differential diagnosis of Alzheimer disease-type dementia. Such kit preferably contains means for the detection of the markers CD5 antigen-like or any of its isoforms, haptoglobin or any of its isoforms and PEDF, respectively. Preferably said means of detection are antibodies that specifically recognize each marker respectively.

The kit can also contain, depending on the particular immunoassay used, suitable labels and other packaged reagents and materials (i.e. wash buffers and the like). Standard immunoassays, such as those described above, can be conducted using these kits.

The kits may be used for the *in vitro* differential diagnosis or monitoring of ATD.

### Differential diagnosis

This invention relates to an *in vitro* method for the diagnosis and discrimination of different ATD. This method is based on a differential diagnosis established by comparing expression levels of biological markers including CD5 antigen-like or any of its isoforms, preferably one or additional markers selected form the group consisting of haptoglobin or any of its isoforms, and PEDF.

The level of expression of said markers in the biological sample of a patient who shows clinical symptoms of an ATD selected from the group consisting of AD, VD, DFT, PiD and LBD, or is suspected with said ATD, is compared to the level of marker expression in subjects who show clinical symptoms of a different ATD selected from the group consisting of AD, VD, DFT, PiD and LBD, or are suspected with said different ATD.

In one embodiment of the invention, a patient suspected with AD is tested for CD5 antigen-like expression. A higher level of CD5 antigen-like expression compared to CD5 antigen-like expression level in patients suspected with FTD or LBD is in favour of AD. Moreover, a higher level of CD5-antigen-like associated with a lower level of haptoglobin expression compared to the respective expression levels of these proteins in patients suspected with FTD is indicative of AD. In a similar manner, a higher level of CD5 antigen-like expression associated with a lower level of PEDF expression compared to the respective expression levels of these proteins in patients suspected with FTD is indicative of AD. A higher level of CD5 antigen-like expression associated with a lower level of expression of both haptoglobin and PEDF compared to the respective expression levels of these proteins in patients suspected with FTD confirms an AD case.

In another embodiment, a patient suspected with FTD is tested for CD5 antigen-like expression. A lower level of CD5 antigen-like expression compared to CD5 antigen-like expression level in subjects suspected with AD or PiD is in favour of FTD. Moreover, a lower level of CD5 antigen-like associated with a higher expression of level of haptoglobin compared to the respective expression levels of these proteins in subjects suspected with AD or PiD is indicative of FTD. In a similar manner, a lower level of CD5 antigen-like expression associated with a higher level of PEDF expression compared to the respective expression levels of these proteins in subjects suspected with AD or PiD is indicative of FTD. A lower level of CD5 antigen-like expression associated with a higher level of expression of both PEDF and haptoglobin, compared to the respective expression levels of these proteins in subjects suspected with AD or PiD confirms a FTD case.

In another embodiment of the invention, a patient suspected with PiD is tested for CD5 antigen-like expression. A higher level of CD5 antigen-like expression compared to CD5 antigen-like expression level in subjects suspected with FTD or LBD is in favour of PiD. Moreover, a higher level of CD5 antigen-like associated with a higher level of expression of haptoglobin compared to the respective expression levels of these proteins in subjects suspected with LBD is indicative of PiD. In a similar manner, a higher level of CD5 antigen-like expression associated with a higher level of PEDF expression compared to the respective expression levels of these proteins in subjects suspected with LBD is indicative of PiD. A higher level of CD5 antigen-like expression associated with a higher level of expression of both PEDF and haptoglobin, compared to the respective expression levels of these proteins in subjects suspected with LBD confirms a PiD case.

In another embodiment, a patient suspected with LBD is tested for CD5 antigen-like expression. A lower level of CD5 antigen-like expression compared to CD5 antigen-like expression level in subjects suspected with AD or PiD is in favour of LBD. Moreover, a lower level of CD5 antigen-like associated with a lower level of expression of haptoglobin compared to the respective expression levels of these proteins in subjects suspected with PiD is indicative of LBD. In a similar manner, a lower level of CD5 antigen-like expression associated with a lower level of PEDF expression compared to the respective expression levels of these proteins in subjects suspected with PiD is indicative of LBD. A lower level of CD5 antigen-like expression associated with a lower level of expression of both PEDF and haptoglobin, compared to the respective expression levels of these proteins in subjects suspected with PiD is very useful to confirm a LBD case.

### Monitoring

This invention further relates to a method for monitoring a patient diagnosed with an Alzheimer's disease. This method comprises determining the level of expression of the CD5 antigen-like or any of its isoforms, in a biological sample of a patient, and comparing it to the level of expression of said marker in a biological sample previously obtained from the same patient.

An increase of the level of expression of CD5 antigen-like or any of its isoforms suggests that the dementia has worsened. Moreover, an increase of the level of expression of the CD5 antigen-like or any of its isoforms associated with an increase of the level of expression of haptoglobin or any of its isoforms, is indicative that the dementia has worsened. An increase of the level of expression of the CD5 antigen-like or any of its isoforms associated with an increase of the level of expression of PEDF is also indicative that the dementia has worsened. In addition, an increase of the level of expression of the three markers, CD5 antigen-like or any of its isoforms, haptoglobin or any of its isoforms and PEDF, is indicative that the dementia has worsened.

The invention is further illustrated by the following example and figures, which do not limit the scope of the invention.

### Legend to figures

***Figure 1*** represents gels of 2D-electrophoresis of proteins present in the cerebrospinal fluid (CSF) of patients suspected with ATD. The proteins were revealed using a silver nitrate staining.

1A, 1B and 1C refer to samples from AD patients. 1D, 1E, 1F and 1G refer to samples from FDT patients. 1H refers to sample from PiD patient. The first number corresponds to the number of the patient in the clinical study and the last number corresponds to the CDR (Clinical Dementia Rating) scale. Thus, for example, patient 23AD1 (Fig. 1A) is the 23^{rd} AD patient in the clinical study and presents an early stage of dementia, whereas patients 54AD3 (Fig. 1C) is the 54^{th} patient of the study and presents a severe dementia.

The lower line underlines the expression of haptoglobin and its isoforms. The median circle or line indicates the expression of CD5 antigen-like or CD5 antigen-like and its isoforms, respectively. The upper circle indicates the PEDF expression.

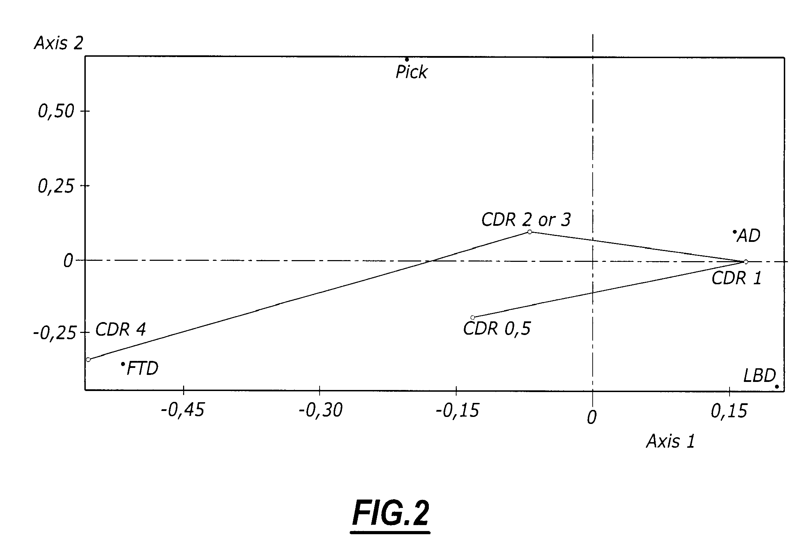

***Figure 2*** is a table that shows the variables used to discriminate ATD. AD refers to Alzheimer's disease, FDT to fronto-temporal dementia, LBD to Lewy's bodies disease and Pick to Pick's disease. CDR relates to Clinical Dementia Rating scale, considering that lower CDR values correspond to the early stages of the dementia.

Axis 1 represents the positive correlation existing between haptoglobin and PEDF. Patients who had higher values for the variables haptoglobin and PEDF are represented on the left of the factorial plane, and patients who had lower values are represented on the right of the plane. Axis 2 is linked to CD5 antigen-like. Patients who had lower values for the CD5 antigen-like are represented in the bottom of the factorial plane, and patients who had higher values are represented in the top of the factorial plane. Units for both axes were arbitrarily chosen.

The groups of diseases scattered in the factorial plane are clearly separated.

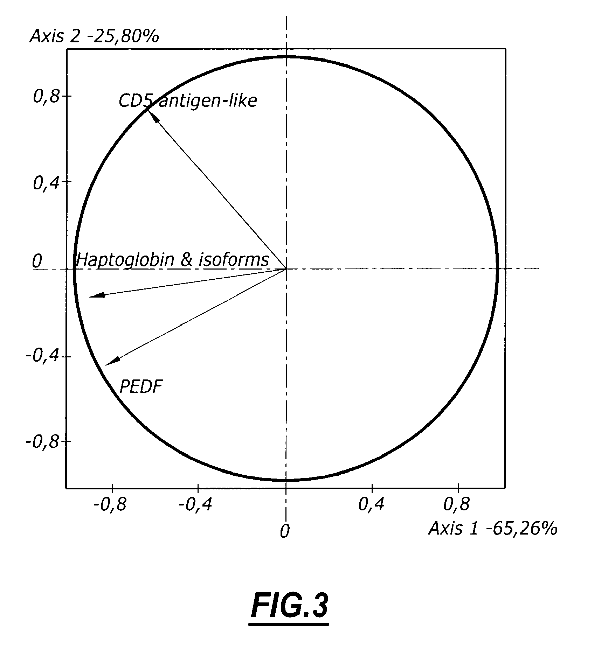

***Figure 3*** is a correlation circle showing more than 90 % of correlation between the different ATD and the specific markers, considering the axes. Axis 1 is linked to haptoglobin and PEDF values and axis 2 is linked to CD5 antigen-like values. Units for both axes were arbitrarily chosen.

### Example

### Materials and methods

**Panel of patients suspected with ATD involved in the experimentation**

Patients showing various clinical symptoms of ATD were roughly classified according to their symptoms, into the following groups:
- 62 patients suspected with AD (including 19 patients suspected with VD)
- 24 patients suspected with FTD;
- 3 patients suspected with LBD;
- 3 patients suspected with PiD;
- 3 patients with other dementia.

A non traumatic lumbar puncture was performed always after informed consent of after a Computerized-tomography (CT) scan. Subjects were placed in a sitting position, under sterile conditions, and a needle was placed in the interspace between vertebrae L4-L5 or L5-S1. Neither specific compressive abnormalities of CSF nor alterations in protein concentration were observed. CSF samples were centrifuged at room temperature for 10 min at 800 g, aliquoted, and immediately frozen in liquid nitrogen and stored at -80°C until use.

### Samples preparation for isoelectrofocusing

An aliquot of 300µL of human CSF was mixed in 600 µL of ice cold acetone and centrifuged at 10000g at 4°C for 10 minutes. The pellet was mixed with 10µL of a solution containing SDS (10% w/v) and DTE (2.3% w/v). The sample was heated at 95°C for 5 min and then diluted with 80 µL of a solution containing 8 M urea, 4% w/v CHAPS, 40 mM TRIS, 65 mM DTE, and a trace of bromophenol blue.

An aliquot of 10µL of human serum was mixed with 10µL of a solution containing SDS (10% w/v) and DTE (2.3% w/v). The sample was heated at 95°C for 5 min and then diluted with 500 µL of a solution containing 8 M urea, 4% w/v CHAPS, 40 mM TRIS, 65 mM DTE, and a trace of bromophenol blue.

### 2D-electrophoresis of proteins present in the CSF

Isoelectric focusing (IEF) was performed on a Multiphor II (Amersham Pharmacia Biotech, Uppsala, Sweden) connected to a refrigerated bath circulator (Multitemp II, Amersham Pharmacia Biotech). Sodium-dodecylsulfate-polyacrylamide gel electrophoresis (SDS-PAGE) was performed in the Protean II xi cell (Bio-Rad) connected to a refrigerated bath circulator (Frigomix R, B. Braun, Germany). Power was supplied by a Multidrive XL (Amersham Pharmacia Biotech) for IEF and a PowerPac 1000 (Bio-Rad) for SDS-PAGE. Gradient gels (160 x 200 x 1.5 mm, 9-16 %) were poured in the Protean II xi multi-gel casting chamber (Bio-Rad). Non-linear immobilized pH gradient strips (IPG pH 3.5-10, Amersham Pharmacia Biotech) were used in the first dimension. 2D-PAGE was essentially as described by Görg et al. (1988) and Bjellquist et al. (1993) for immobilized pH gradients, and by Hochstrasser et al. (1988a, 1988b) and Hochstrasser and Merril (1988) for the improvement of 2D technique and silver nitrate staining. Briefly, IEF was run at 4°C with a linear gradient from 100 to 3500 V until a total of 100 kVh was reached. SDS-PAGE was run at 40 mA/gel at 12°C during 5 h. After silver nitrate staining, gels were scanned on a Personal Densitometer SI (50 µm/pixel, 12 bits/pixel, Molecular Dynamics, Sunnyvale, CA) and subsequently analyzed on a workstation SPARCstation 5 (Sun Microsystems Inc., Moutain View, CA) equipped with the MELANIE II 2D-PAGE software (Bio-Rad), originally developed by Hochstrasser et al. (1991). Identification was performed either by matching spots with known proteins of previously established 2D gels, or by mass spectrometry. In the latter case, glutaraldehyde was omitted in the gel according to Shevchenko et al. (1996). In order to check the reproducibility of our approach, each 2D gel was made twice.

### Results

### Identification of markers useful to discriminate the different ATD

2D-electrophoresis allowed analyzing proteins expression in CSF of patients with ATD. After visual comparison, the profile of expression of three proteins seemed to be different according to the different ATD. These markers were then identified by mass spectroscopy. They correspond to CD5 antigen-like, haptoglobin and PEDF. The results are shown on Figure 1.

Figure 1 revealed the profile of expression of the three markers in AD (1A, 1B, 1C), FTD (1D, 1E, 1F, 1G) and PiD patients (1H). The rosary of spots corresponding to the expression for haptoglobin and its isoforms was well visible (see Fig. 1B). CD5 antigen-like was expressed rather as a unique spot (see Fig. 1A) or as three spots suggesting the existence of at least three isoforms for this protein (see Fig.1H). This pattern seemed to be specific of PiD and could be used as a specific marker for diagnosis. The PEDF expression appeared as a unique spot (see Fig.1 F).

### Correlation between the markers and the ATD

### Principal component analysis

After separation by 2D-electrophoresis, the relative levels of expression of proteins were analysed using Principal Component Analysis (PCA). This method permits to reduce the number of variables by performing a covariance analysis between factors in order to determine relationships between variables (Collins M et al., 2001).

The purpose of this analysis was to establish a correlation between the different ATD and specific biomarkers. With this aim, the inventors determined a small number of linear combinations of the three variables (CD5 antigen-like, haptoglobin, PEDF) that account for most of the variability of the data. They were able to explain as much as 90% of the original data according to two axes (see Fig.2). From this representation, by looking at Axis 1, the inventors have deduced that patients who have higher values for haptoglobin and for PEDF were essentially affected by FTD. Conversely, patients with lower values for haptoglobin and for PEDF were affected by AD or LBD. In addition, by looking at Axis 2, the inventors noticed that FTD and LBD had lower values for CD5 antigen-like, unlike the subjects affected by PiD or even AD.

Based on these results, each group of patients can be differentiated from the others. For example, a patient exhibiting a high level of CD5 antigen-like expression is suspected with AD (or VD) or PiD. If he further shows a low level of haptoglobin and/or PEDF expression, he is expected to be affected with AD. If he further presents a high level of expression of haptoglobin or PEDF, he is supposed to be affected with PiD. A patient presenting a low level of CD5 antigen-like expression is suspected to be affected either with FTD or LBD. If this patient further presents a high level of haptoglobin and/or PEDF expression, he is supposed to be affected with FTD, whereas a low level of haptoglobin and/or PEDF expression is rather associated with LBD. Consequently, a patient with suspicion of an ATD can be tested through such a differential method, which makes it possible to confirm a determined dementia.

The inventors have also studied the Clinical Dementia Rate (CDR) scale. This scale is widely used in longitudinal and clinical studies to gauge Alzheimer's Disease or other neurodegenerative disorders progression (Hughes CP, 1982). In their analysis, the CDR scale appeared strongly linked with the horizontal axis (see Fig.2). The more the disease was worsening (CDR increases), the more the CDR scale moved to the left part of the horizontal axis, suggesting an increase in inflammatory responses.

### Step-by-step discriminating analysis

Further, the proteins spots revealed by 2D-electrophoresis were analysed by discriminating analysis. This approach consists in comparing the volume of identified proteins in comparison with the total quantity of protein present in the whole sample analysed. Quantitative variables are thus identified. Then, using STEPDISC procedure available through SAS/STAT^{®} software (SAS corporation), the number of variables is reduced step-by-step until identification of discriminating markers.

As a result, the inventors have confirmed the relevance of the previously identified markers for the diagnosis of ATD. In particular, they established, with this method, that a high CD5 antigen-like value associated with a low PEDF value allows to discriminate AD from other dementia (p<0,0058).

### REFERENCES

- Andreasen N and Blennow K, (2005) Clin. Neurol. Neurosurg. 107(3):165-73
- Ballard et al. (2004) Am. J. Psychiatry, 61(5) :843-9
- Bjellqvist et al., (1993) Electrophoresis, 14 :1375-78,
- Collins (2001) Advances in neural information processing Systems (NIPS) 14
- Duplan et al., (2001) Neurobiol Aging. 22(1):79-88
- Fladd D, (2005) Geriatr. Nurs. 26(2):117-21
- Görg et al., (1988) Electrophoresis, 9 :531-46,
- Hochstrasser and Merril, (1988) Appl Theor Electrophor 1:35-40,
- Hochstrasser et al., (1991) Clin Comput 8:85-91,
- Hoschstrasser et al., (1988a) Anal Biochem 173 :412-23,
- Hoschstrasser et al., (1988b) Anal Biochem 173 :424-35,
- Hughes CP, (1982) Br J Psychiatry 140:566-72
- Johnson G (1992) Brain Res. Mol. Brain Res. 15(3-4):319-26
- Joseph et al., (2004) Cell 119(2):299-309
- Lippa CF (2004) Am J Alzheimers Dis Other Demen. 19(6):341-4.
- Mc Keith I, (2003) Semin Clin Neuropsychiatry 8(1):46-57
- Sarrias et al. (2004) Tissue antigens, 63(4) :335-44
- Shevchenko et al., (1996) Anal Chem 68:850-58,
- Smeets MB (2003) Cardiovasc. Res. 58(3):689-95
- Tissot et al., (2002) Electrophoresis 23(7-8):1203-6
- Ueno I et al. (2000) Electrophoresis 21(9):1832-45
- Williams GB, et al., (2005) Neuroimage 24(4) :1042-51
- Yamagishi et al. (2004) Med. Hypotheses 63(1) :115-7
- Yamagishi S et al., (2005) Med Hypotheses 64(6):1205-7
- Zlokovic BV et al., (2005) Brain Pathol. 15(1):78-83

## Claims

1. An *in vitro* method for the differential diagnosis of an Alzheimer-type dementia in a patient, which method comprises determining the level of expression of CD5 antigen-like or any of its isoforms in a biological sample of the patient.

2. The method of claim 1, further comprising determining the level of expression of haptoglobin or any of its isoforms in a biological sample of the patient.

3. The method of claim 1 or 2, further comprising determining the level of expression of Pigment Epithelium-Derived Factor.

4. The method of any of claims 1 to 3, wherein the patient shows clinical symptoms of an Alzheimer-type dementia selected from the group consisting of Alzheimer's disease, vascular dementia, fronto-temporal dementia, Pick's disease and Lewy's bodies disease, or is suspected with said Alzheimer-type dementia, and the level of expression of CD5 antigen-like or any of its isoforms in the biological sample of said patient is compared to the level of expression of CD5 antigen-like or any of its isoforms in subjects who show clinical symptoms of a different Alzheimer-type dementia selected from the group consisting of Alzheimer's disease, vascular dementia, fronto-temporal dementia, Pick's disease and Lewy's bodies disease, or are suspected with said different Alzheimer-type dementia.

5. The method of claim 4, wherein a higher level of expression of CD5 antigen-like or any of its isoforms in a biological sample of a patient suspected with Alzheimer's disease, compared to the level of expression of CD5 antigen-like or any of its isoforms in a biological sample of subjects suspected with fronto-temporal dementia or Lewy's bodies disease, is in favour of Alzheimer's disease in said patient.

6. The method of claim 5, wherein a higher level of expression of CD5 antigen-like or any of its isoforms and a lower level of expression of an additional marker selected from the group consisting of haptoglobin or any of its isoforms, and Pigment Epithelium-Derived Factor, compared to the respective expression levels in a biological sample of subjects suspected with fronto-temporal dementia is indicative of Alzheimer's disease in said patient.

7. The method of claim 4, wherein a lower level of expression of CD5 antigen-like or any of its isoforms in a biological sample of a patient suspected with fronto-temporal dementia, compared to the level of expression of CD5 antigen-like or any of its isoforms in a biological sample of subjects suspected with Alzheimer's disease or Pick disease, is in favour of fronto-temporal dementia in said patient.

8. The method of claim 7, wherein a lower level of expression of CD5 antigen-like like or any of its isoforms and a higher level of expression of an additional marker selected from the group consisting of haptoglobin, any of its isoforms, and Pigment Epithelium-Derived Factor, compared to the respective expression levels in a biological sample of subjects suspected with Alzheimer's disease or Pick disease is indicative of fronto-temporal dementia in said patient.

9. The method of claim 4, wherein a higher level of expression of CD5 antigen-like or any of its isoforms in a biological sample of a patient suspected with PiD, compared to the level of expression of CD5 antigen-like or any of its isoforms in a biological sample of subjects suspected with fronto-temporal dementia or Lewy's bodies disease, is in favour of Pick disease in said patient.

10. The method of claim 9, wherein a higher level of expression of CD5 antigen-like or any of its isoforms and a higher level of expression of an additional marker selected from the group consisting of haptoglobin or any of its isoforms, and Pigment Epithelium-Derived Factor, compared to the respective expression levels in a biological sample of subjects suspected with Lewy's bodies disease is indicative of Pick disease in said patient.

11. The method of claim 4, wherein a lower level of CD5 antigen-like expression in a biological sample of a patient suspected with LBD, compared to the level of expression of CD5 antigen-like or any of its isoforms in a biological sample of subjects suspected with Alzheimer disease or Pick disease, is in favour of Pick disease in said patient.

12. The method of claim 11, wherein a lower level of expression of CD5 antigen-like or any of its isoforms and a lower level of expression of an additional marker selected from the group consisting of haptoglobin or any of its isoforms, and Pigment Epithelium-Derived Factor, compared to the respective expression levels in a biological sample of subjects suspected with Pick disease is indicative of Lewy's bodies disease in said patient.

13. The method according to any of claims 1 to 12, wherein the level of expression of the CD5 antigen-like or any of its isoforms is determined by detecting and/or quantifying the CD5 antigen-like protein.

14. The method according to any of claims 1 to 12, wherein the level of expression of the CD5 antigen-like or any of its isoforms is determined by detecting and/or quantifying the CD5 antigen-like mRNA.

15. The method according to any of the preceding claims, wherein the biological sample is cerebrospinal fluid, blood or urine.

16. An *in vitro* method for monitoring a patient diagnosed with an Alzheimer-type dementia, which method comprises determining the level of expression of the CD5 antigen-like or any of its isoforms, in a biological sample of a patient, and comparing it to the level of expression of said marker in a biological sample previously obtained from the same patient, wherein an increase of the level of expression is indicative that the dementia has worsened.

17. The method of claim 16, further comprising determining the level of expression of haptoglobin or any of its isoforms, wherein an increase of the level of expression of both markers is indicative that the dementia has worsened.

18. The method of claim 16, further comprising determining the level of expression of Pigment Epithelium-Derived Factor, wherein an increase of the level of expression of both markers is indicative that the dementia has worsened.

19. The method of claim 16, which comprises determining the level of expression of the three markers CD5 antigen-like or any of its isoforms, haptoglobin or any of its isoforms, and Pigment Epithelium-Derived Factor, wherein an increase of the level of expression of said three markers is indicative that the dementia has worsened.

20. The method of any of claims 16 to 19, wherein the Alzheimer-type dementia is selected from the group consisting of Alzheimer's disease, vascular dementia, fronto-temporal dementia, Pick's disease, and Lewy's bodies disease.

21. A kit useful for differential diagnosis of Alzheimer-type dementia, which comprises:
(i) means for the detection of the level of expression of CD5 antigen-like or any of its isoforms, and
(ii) means for the detection of the level of expression of haptoglobin or any of its isoforms, and/or
(iii) means for the detection of the level of expression of Pigment Epithelium-Derived Factor.

22. The kit of claim 21, which comprises antibodies that specifically recognize CD5 antigen-like or any of its isoforms, haptoglobin or any of its isoforms, and Pigment Epithelium-Derived Factor, respectively.

23. Use of a kit as defined in any of claims 21 or 22 for the *in vitro* differential diagnosis of Alzheimer-type dementia.

24. Use of a kit as defined in any of claims 21 or 22, for the *in vitro* monitoring of a patient diagnosed with an Alzheimer-type dementia.


SEQUENCE LISTING

<110>  INSTITUT NATIONAL DE LA SANTE ET DE LA RECHERCHE MEDICALE

<120>  METHOD FOR THE DIFFERENTIAL DIAGNOSIS AND THE MONITORING OF
       ALZHEIMER-TYPE DEMENTIA

<130>  BET05P0494

<160>  2

<170>  PatentIn version 3.2

<210>  1
<211>  1044
<212>  DNA
<213>  Homo sapiens


<220>
<221>  CDS
<222>  (1)..(1044)

<400>  1
atg gct ctg cta ttc tcc ttg atc ctt gcc att tgc acc aga cct gga
48
Met Ala Leu Leu Phe Ser Leu Ile Leu Ala Ile Cys Thr Arg Pro Gly
1               5                   10                  15

ttc cta gcg tct cca tct gga gtg cgg ctg gtg ggg ggc ctc cac cgc
96
Phe Leu Ala Ser Pro Ser Gly Val Arg Leu Val Gly Gly Leu His Arg
            20                  25                  30

tgt gaa ggg cgg gtg gag gtg gaa cag aaa ggc cag tgg ggc acc gtg
144
Cys Glu Gly Arg Val Glu Val Glu Gln Lys Gly Gln Trp Gly Thr Val
        35                  40                  45

tgt gat gac ggc tgg gac att aag gac gtg gct gtg ttg tgc cgg gag
192
Cys Asp Asp Gly Trp Asp Ile Lys Asp Val Ala Val Leu Cys Arg Glu
    50                  55                  60

ctg ggc tgt gga gct gcc agc gga acc cct agt ggt att ttg tat gag
240
Leu Gly Cys Gly Ala Ala Ser Gly Thr Pro Ser Gly Ile Leu Tyr Glu
65                  70                  75                  80

cca cca gca gaa aaa gag caa aag gtc ctc atc caa tca gtc agt tgc
288
Pro Pro Ala Glu Lys Glu Gln Lys Val Leu Ile Gln Ser Val Ser Cys
                85                  90                  95

aca gga aca gaa gat aca ttg gct cag tgt gag caa gaa gaa gtt tat
336

Thr Gly Thr Glu Asp Thr Leu Ala Gln Cys Glu Gln Glu Glu Val Tyr
            100                 105                 110

gat tgt tca cat gat gaa gat gct ggg gca tcg tgt gag aac cca gag
384
Asp Cys Ser His Asp Glu Asp Ala Gly Ala Ser Cys Glu Asn Pro Glu
        115                 120                 125

agc tct ttc tcc cca gtc cca gag ggt gtc agg ctg gct gac ggc cct
432
Ser Ser Phe Ser Pro Val Pro Glu Gly Val Arg Leu Ala Asp Gly Pro
    130                 135                 140

ggg cat tgc aag gga cgc gtg gaa gtg aag cac cag aac cag tgg tat
480
Gly His Cys Lys Gly Arg Val Glu Val Lys His Gln Asn Gln Trp Tyr
145                 150                 155                 160

acc gtg tgc cag aca ggc tgg agc ctc cgg gcc gca aag gtg gtg tgc
528
Thr Val Cys Gln Thr Gly Trp Ser Leu Arg Ala Ala Lys Val Val Cys
                165                 170                 175

cgg cag ctg gga tgt ggg agg gct gta ctg act caa aaa cgc tgc aac
576
Arg Gln Leu Gly Cys Gly Arg Ala Val Leu Thr Gln Lys Arg Cys Asn
            180                 185                 190

aag cat gcc tat ggc cga aaa ccc atc tgg ctg agc cag atg tca tgc
624
Lys His Ala Tyr Gly Arg Lys Pro Ile Trp Leu Ser Gln Met Ser Cys
        195                 200                 205

tca gga cga gaa gca acc ctt cag gat tgc cct tct ggg cct tgg ggg
672
Ser Gly Arg Glu Ala Thr Leu Gln Asp Cys Pro Ser Gly Pro Trp Gly
    210                 215                 220

aag aac acc tgc aac cat gat gaa gac acg tgg gtc gaa tgt gaa gat
720
Lys Asn Thr Cys Asn His Asp Glu Asp Thr Trp Val Glu Cys Glu Asp
225                 230                 235                 240

ccc ttt gac ttg aga cta gta gga gga gac aac ctc tgc tct ggg cga
768
Pro Phe Asp Leu Arg Leu Val Gly Gly Asp Asn Leu Cys Ser Gly Arg
                245                 250                 255

ctg gag gtg ctg cac aag ggc gta tgg ggc tct gtc tgt gat gac aac
816
Leu Glu Val Leu His Lys Gly Val Trp Gly Ser Val Cys Asp Asp Asn
            260                 265                 270

tgg gga gaa aag gag gac cag gtg gta tgc aag caa ctg ggc tgt ggg
864
Trp Gly Glu Lys Glu Asp Gln Val Val Cys Lys Gln Leu Gly Cys Gly

275                 280                 285

aag tcc ctc tct ccc tcc ttc aga gac cgg aaa tgc tat ggc cct ggg
912
Lys Ser Leu Ser Pro Ser Phe Arg Asp Arg Lys Cys Tyr Gly Pro Gly
    290                 295                 300

gtt ggc cgc atc tgg ctg gat aat gtt cgt tgc tca ggg gag gag cag
960
Val Gly Arg Ile Trp Leu Asp Asn Val Arg Cys Ser Gly Glu Glu Gln
305                 310                 315                 320

tcc ctg gag cag tgc cag cac aga ttt tgg ggg ttt cac gac tgc acc
1008
Ser Leu Glu Gln Cys Gln His Arg Phe Trp Gly Phe His Asp Cys Thr
                325                 330                 335

cac cag gaa gat gtg gct gtc atc tgc tca gga tag
1044
His Gln Glu Asp Val Ala Val Ile Cys Ser Gly
            340                 345


<210>  2
<211>  347
<212>  PRT
<213>  Homo sapiens

<400>  2

Met Ala Leu Leu Phe Ser Leu Ile Leu Ala Ile Cys Thr Arg Pro Gly
1               5                   10                  15


Phe Leu Ala Ser Pro Ser Gly Val Arg Leu Val Gly Gly Leu His Arg
            20                  25                  30


Cys Glu Gly Arg Val Glu Val Glu Gln Lys Gly Gln Trp Gly Thr Val
        35                  40                  45


Cys Asp Asp Gly Trp Asp Ile Lys Asp Val Ala Val Leu Cys Arg Glu
    50                  55                  60


Leu Gly Cys Gly Ala Ala Ser Gly Thr Pro Ser Gly Ile Leu Tyr Glu
65                  70                  75                  80


Pro Pro Ala Glu Lys Glu Gln Lys Val Leu Ile Gln Ser Val Ser Cys
                85                  90                  95


Thr Gly Thr Glu Asp Thr Leu Ala Gln Cys Glu Gln Glu Glu Val Tyr
            100                 105                 110

Asp Cys Ser His Asp Glu Asp Ala Gly Ala Ser Cys Glu Asn Pro Glu
        115                 120                 125

Ser Ser Phe Ser Pro Val Pro Glu Gly Val Arg Leu Ala Asp Gly Pro
    130                 135                 140

Gly His Cys Lys Gly Arg Val Glu Val Lys His Gln Asn Gln Trp Tyr
145                 150                 155                 160

Thr Val Cys Gln Thr Gly Trp Ser Leu Arg Ala Ala Lys Val Val Cys
                165                 170                 175

Arg Gln Leu Gly Cys Gly Arg Ala Val Leu Thr Gln Lys Arg Cys Asn
            180                 185                 190

Lys His Ala Tyr Gly Arg Lys Pro Ile Trp Leu Ser Gln Met Ser Cys
        195                 200                 205

Ser Gly Arg Glu Ala Thr Leu Gln Asp Cys Pro Ser Gly Pro Trp Gly
    210                 215                 220

Lys Asn Thr Cys Asn His Asp Glu Asp Thr Trp Val Glu Cys Glu Asp
225                 230                 235                 240

Pro Phe Asp Leu Arg Leu Val Gly Gly Asp Asn Leu Cys Ser Gly Arg
                245                 250                 255

Leu Glu Val Leu His Lys Gly Val Trp Gly Ser Val Cys Asp Asp Asn
            260                 265                 270

Trp Gly Glu Lys Glu Asp Gln Val Val Cys Lys Gln Leu Gly Cys Gly
        275                 280                 285

Lys Ser Leu Ser Pro Ser Phe Arg Asp Arg Lys Cys Tyr Gly Pro Gly
    290                 295                 300

Val Gly Arg Ile Trp Leu Asp Asn Val Arg Cys Ser Gly Glu Glu Gln
305                 310                 315                 320

Ser Leu Glu Gln Cys Gln His Arg Phe Trp Gly Phe His Asp Cys Thr
                325                 330                 335

His Gln Glu Asp Val Ala Val Ile Cys Ser Gly
            340                     345